# EUROPEAN PATENT APPLICATION

(11) **EP 4 389 083 A1**
(43) Date of publication of application: **26.06.2024**
(21) Application number: 23219853.1
(22) Date of filing: 22.12.2023
(51) Int. Cl.: A61F 2/38

(54) **ANTI-LUXATION KNEE PROSTHESIS ASSEMBLY**

(30) Priority: 22.12.2022 US 202263434584 P
(71) Applicant: Zimmer, Inc., Warsaw, IN 46580 (US)
(72) Inventor: YOKO, Tim, Granger, 46530 (US); BARKER, Joshua, Warsaw, 46582 (US); REIDY, John, Warsaw, 46580 (US); VANDIEPENBOS, Jeffery A., New Paris, 46553 (US); BLAYLOCK, Jeff, Fort Wayne, 46807 (US); MEADOWS, David, Warsaw, 46580 (US); BYRD, Brian D., North Webster, 46555 (US)
(74) Representative: Taor, Simon Edward William

(57) **Abstract**

A prosthesis assembly may include a femoral component, a tibial bearing component engaged by the femoral component and a tibial baseplate. The prosthesis assembly may include a hinge post coupled to the femoral component and at least partially received in a recess in the tibial baseplate. The prosthesis assembly may include a bushing received in a recess in the tibial baseplate. The hinge post is at least partially received by the bushing and the bushing includes one or more engagement features configured to couple the bushing to the hinge post.

## Description

### FIELD

The present subject matter relates to orthopedic prostheses and, more particularly, to orthopedic prosthesis used in constrained knee arthroplasties.

### BACKGROUND

Orthopedic procedures and prostheses are commonly utilized to repair and/or replace damaged bone and tissue in the human body. Generally, the knee is formed by the pair of condyles at the distal portion of the femur, the lower surfaces of which bear upon the correspondingly shaped proximal surface plateau of the tibia. The femur and tibia are connected by means of ligaments such as, the posterior cruciate ligament, the lateral collateral ligament, the medial collateral ligament, and the anterior cruciate ligament. These ligaments provide stability to the knee joint.

Prosthetic knee joints can be considered either constrained or unconstrained. For the purposes of this discussion, constrained prosthetic knee systems include femoral and tibial prostheses, which are mechanically linked or constrained to each other to limit relative movement between the femoral and tibial prostheses. Common mechanisms for such mechanical linkage is by a hinge, band or other linkage structure. An unconstrained prosthetic knee system includes femoral and tibial prostheses which are not mechanically linked. An unconstrained knee utilizes the patient's existing ligaments and other soft tissue to provide joint stability. With this in mind, constrained prosthetic knees have particular applicability to cases in which a patient has experienced ligament loss and/or the existing ligaments do not provide adequate support and stability to the knee.

Various constrained knee designs are known. One such design includes a hinge post. This hinge post configuration is positioned within a tibial baseplate (with an end protruding therefrom) and is connected to the femoral component. One hinge post configuration is the NexGen^{®} Rotating Hinge Knee owned by the applicant, for example.

### OVERVIEW

This disclosure pertains generally to improved constrained knee prostheses, particularly those utilizing a hinge post. Some constrained knee prostheses with hinge posts utilize a design where the femoral component (and hinge post) are free to move generally proximal/distal relative to the tibial baseplate and the tibial bearing component. Such arrangement can allow for distraction of the knee j oint. However, the present inventors have recognized that a certain segment of patients receiving a constrained knee prosthesis with the hinge post may have insufficient soft tissue in the knee joint to prevent distraction and then luxation of the femoral component from the tibial baseplate and the tibial bearing component. Luxation can result in pain and other complications for the patient.

Thus, the present inventors have recognized that for the certain segment of the patients with insufficient soft tissue, distraction of the femoral component from the tibial baseplate and the tibial bearing component should be limited. The present inventors recognize various techniques and apparatuses such as a capture element that can interact with the hinge post and/or a bushing of the constrained knee prostheses to limit distraction. As used herein the terms "limiting distraction", "limit distraction", "limits distraction", "limited distraction" or the like includes various prosthesis configurations as further discussed herein. For example, with one example of limited distraction, the femoral component is capable of a certain degree of proximal/distal movement (e.g., movement of a few millimeters to a few centimeters) relative to the tibial baseplate and the tibial bearing component. However, such degree of movement is eventually restricted/halted/stopped such that the femoral component is not capable of further proximal/distal movement such as proximal/distal movement that could result in luxation of the femoral component from the tibial baseplate and the tibial bearing component. This configuration for the prosthesis should be contrasted with configurations of the prosthesis that are capable of "full distraction" where proximal/distal movement of the femoral component relative to the tibial baseplate and the tibial bearing component is not eventually restricted/halted/stopped by a component of the prosthesis and luxation of the knee joint can result if insufficient soft tissue is present. The terms "limiting distraction", "limit distraction", "limits distraction", "limited distraction" or the like includes prosthesis configurations where the proximal/distal movement of the femoral component is substantially entirely limited save for micromotion. The term "micromotion" refers to the small motions that may exist between prosthesis components, such as between the tibial baseplate and capture element, respectively, upon application of force. Such small motions may occur as a result of material deformation in one or both of the interacting components, or may result from slight spaces or clearances therebetween, for example. Micromotion is distinguished from larger movements of components such as the proximal/distal movement of the femoral component relative to the tibial baseplate and the tibial bearing component.

Additional features and benefits of the various examples provided herein will be discussed and/or will be apparent to one of ordinary skill in the art.

To further illustrate the apparatuses, systems and methods disclosed herein, the following non-limiting examples are provided, and which are referred to below as techniques. Parts or all of these examples/techniques can be combined in any manner.

In some aspects, the techniques described herein relate to a prosthesis assembly for a constrained knee including: a femoral component; a tibial bearing component engaged by the femoral component; a tibial baseplate having a distal surface, a proximal surface opposite the distal surface and facing the tibial bearing component, a periphery extending between the proximal surface and the distal surface and a keel extending distally from the distal surface; a hinge post coupled to the femoral component and at least partially received in a recess in the tibial baseplate; and a bushing received in a recess in the tibial baseplate, wherein the hinge post is at least partially received by the bushing, and wherein the bushing includes one or more engagement features configured to couple the bushing to the hinge post.

In some aspects, the techniques described herein relate to a prosthesis assembly, further including a capture element coupled to one of a shackle or the tibial baseplate, wherein the capture element is engaged by at least one of the bushing or the hinge post to limit distraction of the femoral component from the tibial bearing component and the tibial baseplate.

In some aspects, the techniques described herein relate to a prosthesis assembly, wherein at least one of: the capture element is spaced a distance proximal of the bushing, and wherein the distance allows for a limited degree of distraction of the femoral component from the tibial bearing component and tibial baseplate or the capture element is proximal to and abuts a proximal end of the bushing and thereby entirely limits distraction of the femoral component from the tibial bearing component and tibial baseplate.

In some aspects, the techniques described herein relate to a prosthesis assembly, wherein the capture element includes one of a nut configured to thread to the shackle or a retention clip insertable into a receptacle within the tibial baseplate.

In some aspects, the techniques described herein relate to a prosthesis assembly, wherein the receptacle communicates with the recess and has an opening at the periphery of the tibial baseplate configured to receive the retention clip.

In some aspects, the techniques described herein relate to a prosthesis assembly, wherein the bushing is configured to form an snap-fit to capture the hinge post.

In some aspects, the techniques described herein relate to a prosthesis assembly, wherein, during insertion of the hinge post into the recess, the bushing is configured to temporarily deflect outward into an aperture in an inner wall that forms at a portion of the recess of the tibial baseplate.

In some aspects, the techniques described herein relate to a prosthesis assembly, wherein the hinge post includes one or more projections configured to force the bushing to temporarily deflect outward into the aperture.

In some aspects, the techniques described herein relate to a prosthesis assembly 7 or 8, wherein the hinge post includes a groove configured to receive one or more inward extending projections of the bushing, wherein the one or more inward extending projections extend toward a center of the hinge post.

In some aspects, the techniques described herein relate to a prosthesis assembly, wherein the bushing includes a first bushing configured to receive a proximal portion of the hinge post within a proximal portion of the recess and a second bushing configured to receive a distal portion of the hinge post within a portion of distal portion of the recess, and wherein the first bushing is spaced from the second bushing.

In some aspects, the techniques described herein relate to a prosthesis assembly, wherein the hinge post includes one or more retention features configured to be engaged by the bushing to limit distraction of the femoral component from the tibial bearing component and tibial baseplate.

In some aspects, the techniques described herein relate to a prosthesis assembly, wherein the hinge post is coupled to the bushing by first threads that couple with second threads of the bushing, and wherein coupling of the first threads with the second threads positions the bushing a desired location with respect to a capture element.

In some aspects, the techniques described herein relate to a prosthesis system for a constrained knee including: a femoral component; a tibial bearing component configured to articulate with the femoral component; a tibial baseplate having a distal surface, a proximal surface opposite the distal surface and facing the tibial bearing component, a periphery extending between the proximal surface and the distal surface and a keel extending distally from the distal surface; a hinge post coupled to the femoral component and at least partially received in a recess in the tibial baseplate; a bushing received in a recess in the tibial baseplate, wherein the hinge post is at least partially received by the bushing, and wherein the bushing includes one or more engagement features configured to couple the bushing to the hinge post; and a retention clip configured to couple with the tibial baseplate and configured to allow at least a portion of the hinge post to pass therethrough, wherein, when coupled to the tibial baseplate, the retention clip is configured to be engaged by the bushing to limit distraction of the femoral component from the tibial bearing component and tibial baseplate.

In some aspects, the techniques described herein relate to a prosthesis system, wherein the retention clip is insertable into a receptacle within the tibial baseplate, and is configured to engage a wall of the receptacle in an interference fit.

In some aspects, the techniques described herein relate to a prosthesis system, wherein the receptacle communicates with the recess and has an opening at the periphery of the tibial baseplate configured to receive the retention clip.

In some aspects, the techniques described herein relate to a prosthesis system, wherein the bushing is configured to form an snap-fit to capture the hinge post, wherein the bushing has a thru hole configured to allow at least a portion of the hinge post to pass through the bushing, wherein, during insertion of the hinge post into the recess: the bushing is configured to temporarily deflect outward into an aperture in an inner wall that forms at a portion of the recess of the tibial baseplate and the hinge post includes one or more projections configured to force the bushing to temporarily deflect outward into the aperture.

In some aspects, the techniques described herein relate to a prosthesis system, wherein the hinge post includes a groove configured to receive one or more inward extending projections of the bushing, wherein the one or more inward extending projections extend toward a center of the thru hole.

In some aspects, the techniques described herein relate to a method of assembling a prosthesis assembly for a constrained knee, the method including: coupling a hinge post with a femoral component; inserting a bushing while receiving at least a portion of the hinge post into a recess in a tibial baseplate; coupling a capture element to the tibial baseplate; coupling the hinge post with the bushing within the recess; coupling a tibial bearing component to the tibial baseplate; and mounting the femoral component on the tibial bearing component.

In some aspects, the techniques described herein relate to a method, wherein coupling the hinge post with the bushing within the recess of the tibial baseplate includes temporarily deflecting the bushing outward to allow for passage of the hinge post along the bushing and engaging the bushing with one or more retention features of the hinge post.

In some aspects, the techniques described herein relate to a method, wherein coupling the capture element to the tibial baseplate includes positioning the capture element adjacent the recess of the tibial baseplate and includes inserting the capture element through an opening in a periphery of the tibial baseplate into a receptacle that is adjacent to and communicates with the recess.

In some aspects, the techniques described herein relate to a method, wherein coupling the hinge post with the bushing within the recess positions the bushing at a desired location relative to the capture element.

In some aspects, the techniques described herein relate to a method, further including engaging the capture element with the bushing or the hinge post to limit distraction of the femoral component from the tibial bearing component and tibial baseplate.

### BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various examples discussed in the present document.
FIGS. 1 and 2 illustrate knee joint structures providing suitable environments in which the constrained prosthesis assemblies in accordance with an example of the present application can be utilized.
FIG. 3 is a perspective view of a constrained knee prosthesis assembly according to an example of the present application.
FIG. 3A is a perspective view of the constrained knee prosthesis assembly of FIG. 3 undergoing distraction with a femoral component moving proximal/distal movement relative to a tibial baseplate and a tibial bearing component according to an example of the present application.
FIG. 4 is an exploded view of the constrained knee prosthesis assembly of FIG. 3.
FIG. 5 is a cross-sectional view of a second example of a constrained knee prosthesis assembly with a capture element comprising a retention clip configured to limit distraction of the femoral component relative to the tibial baseplate and/or the tibia bearing component according to an example of the present application.
FIG. 6 is a top perspective view of the tibial baseplate and the capture element of FIG. 5 being moved into a desired position within a receptacle of the tibial baseplate to limit distraction according to an example of the present application.
FIG. 7 is a top plan view of the capture element (the retention clip) of FIGS. 5 and 6 inserted into the receptacle so as to block portions of the recess and a bushing on a proximal end thereof according to an example of the present application
FIG. 8 is a cross-sectional view of the femoral component, the tibial baseplate and the tibial bearing component of a constrained knee prosthesis assembly similar to that of FIG. 5 but using another example of a bushing that is snap-fit connection to the hinge post within at least a portion of a recess of the tibial baseplate according to an example of the present application.
FIGS. 9A-9C illustrate a process of how the hinge post and the bushing are inserted into the recess of the tibial baseplate and coupled together to form the snap-fit connection of FIG. 8 according to an example of the present application.
FIG. 10 shows a perspective view of the first and second pieces of the bushing and the hinge post of FIGS. 8 and 9C in isolation showing interaction of engagement features according to an example of the present application.
FIG. 11 is a cross-sectional view of the tibial baseplate and the tibial bearing component of another constrained knee prosthesis assembly with a hinge post and a bushing having a modified configuration as compared with previous examples.
FIG. 12 is a cross-sectional view of the femoral component, the tibial baseplate and the tibial bearing component of yet another constrained knee prosthesis assembly with a different example of a capture element (a nut) coupled to the shackle and a hinge post and bushing having a modified configuration as compared with prior examples according to an example of the present application.
FIG. 13 is a perspective view of the hinge post of FIG. 12 illustrating a plurality of barbs positioned in a spaced proximal/distal relationship and staggered circumferential relationship to one another according to an example of the present application.

### DETAILED DESCRIPTION

The present application relates to constrained tibial prosthesis assemblies and systems including tibial baseplates, capture elements, bushings and hinge posts among other components. This application focuses on limiting distraction of the femoral component relative to the tibial baseplate and/or the tibial bearing component through various features, techniques and component including the capture elements discussed herein. Limiting distraction can prevent luxation of the knee joint and corresponding pain, discomfort and a possible need for medical intervention.

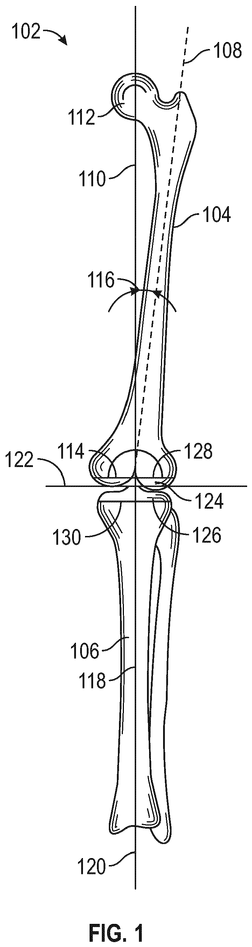

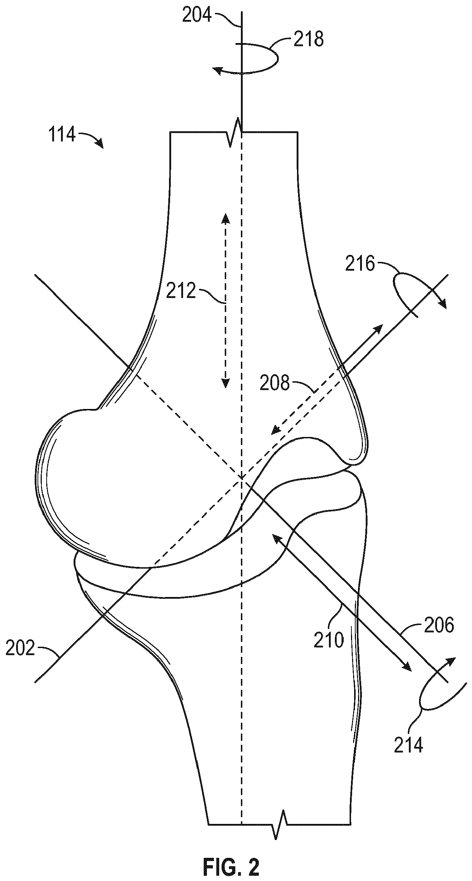

To better understand knee joint replacement procedures, it can be helpful to understand the relationship of bones and bone cuts that can be made to orient various provisional and permanent prosthesis components within a knee joint. FIGS. 1 and 2 illustrate several features of knee joint structures and orientations. In FIG. 1, a frontal view of a lower limb 102, including a femur 104 and a tibia 106, is shown to illustrate various lower limb axes. The femur 104 has an anatomic axis 108 that coincides generally with its intramedullary canal. The femur 104 also has a mechanical axis 110, or load axis, running from the center of a femoral head 112 to the center of a knee joint 114. The angle 116 extending between these two axes varies among the patient population, but is generally on the order of between 5-7 degrees, inclusive. Like the femur 104, the tibia 106 also has an anatomic axis coinciding generally with its intramedullary canal. The mechanical axis 118 of the tibia 106 runs from the center of the knee joint 114 to the center of an ankle region 120 and is generally collinear with its anatomic axis.

A joint line 122, about which the knee joint 114 flexes, is approximately parallel to a line through medial and lateral femoral condyles 124 and to a tibial plateau 126. Although illustrated as perpendicular in FIG. 1, the joint line 122 can extend at a varus or valgus angle relative to the mechanical axes 110 and 118 of the femur 104 and tibia 106, respectively. Normally, during a partial or total knee replacement procedure, portions of a distal end of the femur 104 or a proximal end of the tibia 106 are resected to be parallel or approximately parallel to the joint line 122, and thus perpendicular to the mechanical axes 110 and 118, as indicated at 128 and 130, respectively.

FIG. 2 illustrates a closer view of the knee joint 114 and its coordinate system, in which a medial/lateral axis 202 corresponds approximately to the joint line 122 (FIG. 1), a proximal/distal axis 204 corresponds approximately to the mechanical axes 110 and 118 (FIG. 1), and an anterior/posterior axis 206 is approximately normal to the other two axes. Position along each of these axes can be depicted by arrows, which can represent the medial/lateral 208, anterior/posterior 210, and proximal/distal 212 positioning of inserted prosthesis components. Rotation about each of these axes can also be depicted by arrows. Rotation about the proximal/distal axis 204 can correspond anatomically to external rotation of a femoral component, while rotation about the anterior/posterior axis 206 and medial/lateral axis 202 can correspond to extension plane slope and varus/valgus angle of a component, respectively. Depending on a position of the proximal tibial cut 130 (FIG. 1) made, a varus/valgus angle 214, extension plane angle 216, external rotation 218, or joint extension gap can be affected. Similarly, a position of the distal femoral cut 128 (FIG. 1) can affect the location of the joint line 122, the extension gap, the varus/valgus angle 214, or the extension plane angle 216.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the use of the terms "proximal" and "distal" should be interpreted as though the patient were standing with the knee joint in extension. The intent is to differentiate the terms "proximal" and "distal" from the terms "anterior" and "posterior". As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior". Similarly, the term "lateral" refers to the opposite direction of "medial". The term "medial/lateral" means medial to lateral or lateral to medial. The term "proximal/distal" means proximal to distal or distal to proximal. The term "anterior/posterior" means anterior to posterior or posterior to anterior.

As used herein, the "periphery" of a tibial baseplate refers to any periphery as viewed in a top plan view, e.g., in a generally transverse anatomical plane. Alternatively, the periphery of a tibial baseplate may be any periphery as viewed in bottom plan view, e.g., in a generally transverse plane and looking at the distal surface adapted to contact a resected proximal surface of a tibial bone.

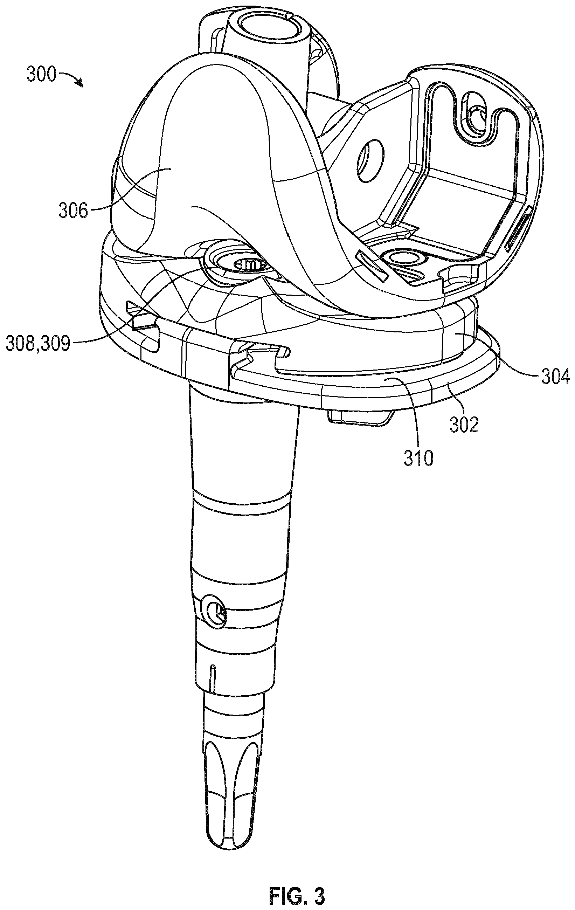

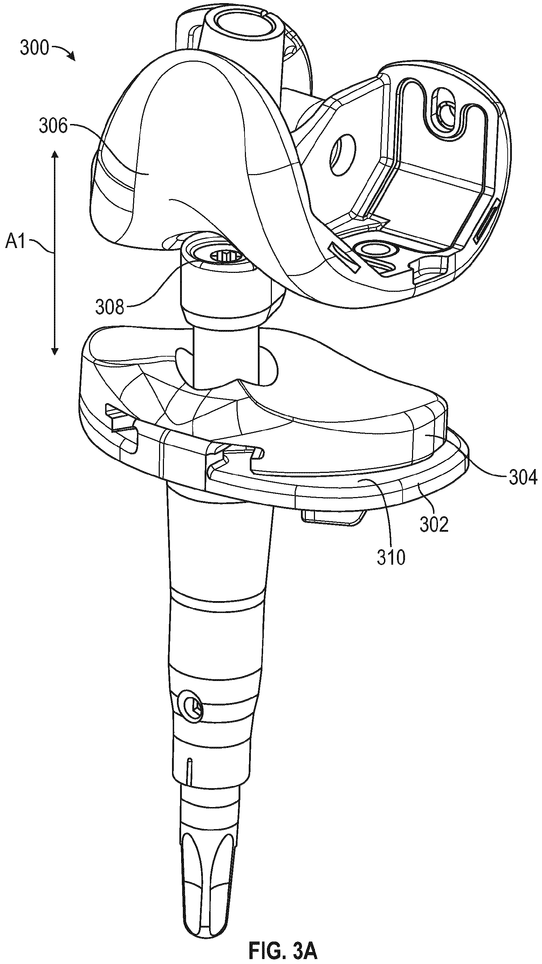

FIG. 3 shows a prosthesis assembly 300 for a constrained knee. The prosthesis assembly 300 can include a tibial baseplate 302, a tibial bearing component 304 (sometime called a meniscal component, poly, articular component or bearing), a femoral component 306 and a hinge post 308.

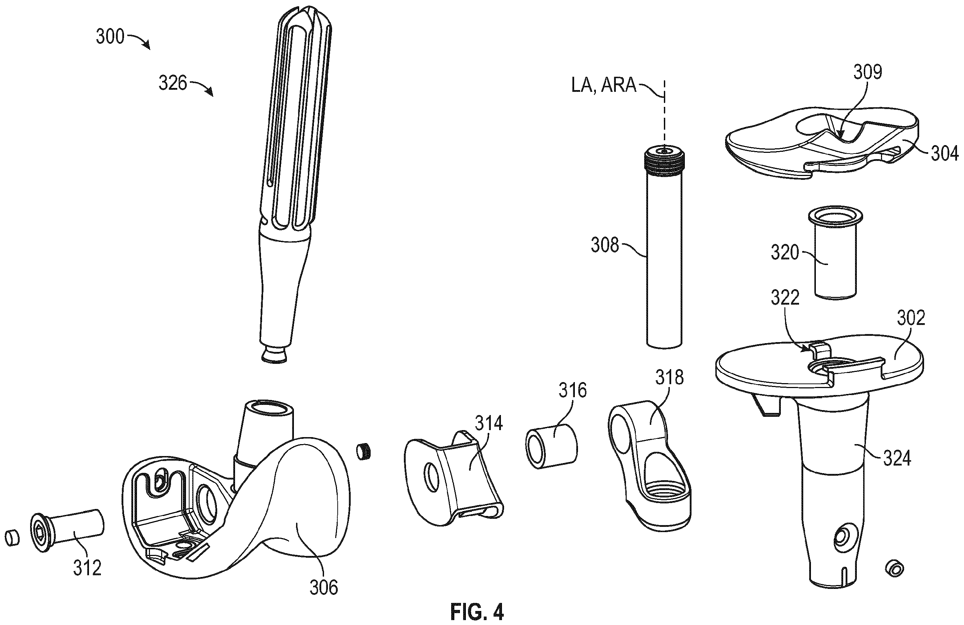

The tibial bearing component 304 can be coupled to and can be positioned atop a proximal surface 310 of the tibial baseplate 302. The tibial bearing component 304 can be formed of polymer material such as Ultra-High-Molecular-Weight-Polyethylene ("UHMWPE"), etc. The tibial bearing component 304 can be configured to articulate with the femoral component 306 through knee joint flexion and extension as known in the art. The prosthesis assembly 300 has the femoral component 306 and the tibial baseplate 302 mechanically linked to one another. This is accomplished by the hinge post 308 and other components further illustrated and discussed in FIG. 4. The hinge post 308 is coupled to the femoral component 306 and is received within a recess 309 of the tibial bearing component 304 and a recess 322 (FIG. 4) of the tibial baseplate 302.

FIG. 3A shows distraction of the knee joint. In particular, the femoral component 306 is moved generally proximal/distal (as indicated by arrow A1) from the tibial bearing component 304 and the tibial baseplate 302. Luxation of the knee joint can result if insufficient soft tissue remains in the knee joint to retain the femoral component 306 on the tibial bearing component 304 in the manner shown in FIG. 3. Movement between the femoral component 306 and the tibial bearing component 304 and the tibial baseplate 302 creates a distance (gap) between the femoral component 306 and an articular surface of the tibial bearing component 304. As the hinge post 308 remains at least partially retained within recesses of the tibial baseplate 302 and/or the tibial bearing component 304 in the example of FIG. 3A, distraction of the femoral component 306 from the tibial bearing component 304 and the tibial baseplate 302 is generally along the proximal/distal axis 204 and along the direction 212 as indicated in FIG. 2. However, should the hinge post 308 come free of the recesses 309 and 322 (FIG. 4), a luxation of the knee joint can result. Luxation can be painful and can result in other complications for the patient.

FIG. 4 shows an exploded view of the prosthesis assembly 300, the tibial baseplate 302, the tibial bearing component 304, the femoral component 306, the hinge post 308 and further illustrates a hinge axle 312, poly box 314, axle bushing 316, shackle 318 and a bushing 320.

The hinge post 308 is connected to femoral component 306 via the shackle 318, the axle bushing 316 and the hinge axle 312. A distal portion of the shackle 318 is received in the recess 309 in the tibial bearing component 304 and the distal portion is threaded or otherwise connected to the hinge post 308. The hinge post 308 extends distally through the recess 309 of the tibial bearing component 304 and is received in a recess 322 of the tibial baseplate 302. The recess 322 of the tibial baseplate 302 that receives the hinge post 308 can at least partially be formed by a keel 324 of the tibial baseplate 302. The hinge post 308 can be moveable (e.g., rotatable and/or capable of distraction as shown in FIG. 3A) relative to the tibial bearing component 304 and/or the tibial baseplate 302. The hinge post 308 can be rotatably connected to femoral component 306 via the hinge axle 312. Thus, a longitudinal axis LA that defines a centerline of the hinge post 308 can define an axis of rotation/articulation ARA for the knee joint as the femoral component 306 and the tibial baseplate 302 are mechanically linked.

When assembled, the shackle 318 can be placed between opposing walls of poly box 314. When assembled on the hinge axle 312, the axle bushing 316 additionally resides within an aperture on a proximal portion of the shackle 318. The shackle 318 and hinge post 308 can be formed from suitable materials such as a titanium alloy, a cobalt-chromium alloy, etc. while the axle bushing 316 and the poly box 314 can be formed from a different materials such as plastic, e.g., UHMWPE. The axle bushing 316 acts as a bearing between the shackle 318 and the hinge axle 312. The poly box 314 acts as a bearing between the femoral component 306 and the shackle 318.

The prosthesis assembly 300 of FIG. 4 shows a system 326 of components where distraction of the knee is not limited by a capture element or other feature of the prosthesis assembly 300. Thus, the system 326 provides components that when assembled are configured for full distraction. Thus, soft tissue of the knee is relied upon to limit distraction between the femoral component 306 and the tibial baseplate 302 and the tibial bearing component 304 as discussed previously. The bushing 320 can be configured to insert into the recess 322 of at least the tibial baseplate 302. The bushing 320 can also insert into or through the recess 309 (FIG. 3) of the tibial bearing component 304 in some examples. The bushing 320 can be configured to receive at least a portion of the hinge post 308. The bushing 320 can act as a bearing between the hinge post 308 and the tibial baseplate 302. The hinge post 308 can be moveable generally proximal/distal relative to the bushing 320 such as during the distraction portrayed in FIG. 3A.

Determining if full joint distraction is desirable based upon the patient anatomy. This determination process can include ascertaining a condition, functionality and amount of soft tissue in the knee joint including the number and condition of any remaining ligaments. The determination process can also include the physician distracting the knee joint, performing a range of motion of the knee joint, measuring gaps between bone and/or soft tissue within the knee joint such as measuring the joint extension gap discussed in reference to FIGS. 1 and 2, or the like. Some or all of these determinations can be performed prior to or after implantation of femoral component, the tibial bearing component, the tibial baseplate and/or other components.

If the physician determines that, based upon the patient anatomy, the knee joint is sufficient to maintain the femoral component mechanically linked to the tibial baseplate (e.g., a luxation of the knee joint will likely not result) during full distraction, the physician can implement a first constrained prosthesis assembly configured to allow for full distraction of the femoral component from the tibial bearing component and/or the tibial baseplate. Alternatively, if the physician determines that, based upon the patient anatomy, the knee joint is insufficient to maintain the femoral component mechanically linked to the tibial baseplate (e.g., a luxation of the knee joint could possibly result) during full distraction, the physician can implement a second constrained prosthesis assembly configured to allow for limited distraction of the femoral component from the tibial bearing component and/or the tibial baseplate.

According to one example, a method of assembling a prosthesis assembly for a constrained knee is discussed and shown herein. The method can include: coupling a hinge post with a femoral component, inserting a bushing while receiving at least a portion of the hinge post into a recess in a tibial baseplate, coupling a capture element to the tibial baseplate, coupling the hinge post with the bushing within the recess, coupling a tibial bearing component to the tibial baseplate and mounting the femoral component on the tibial bearing component.

The method can optionally include that the coupling the hinge post with the bushing within the recess of the tibial baseplate can include temporarily deflecting the bushing outward to allow for passage of the hinge post along the bushing and engaging the bushing with one or more retention features of the hinge post. The coupling the capture element to the tibial baseplate can include positioning the capture element adjacent the recess of the tibial baseplate and includes inserting the capture element through an opening in a periphery of the tibial baseplate into a receptacle that is adjacent to and communicates with the recess. The coupling the hinge post with the bushing within the recess can position the bushing at a desired location relative to the capture element. The method can further include engaging the capture element with the bushing or the hinge post to limit distraction of the femoral component from the tibial bearing component and tibial baseplate.

It should be noted that the step of coupling a tibial bearing component to the tibial baseplate and/or mounting the femoral component on the tibial bearing component can be performed prior to or after the steps of coupling a hinge post with a femoral component, inserting a bushing into a recess in a tibial baseplate while receiving at least a portion of the hinge post into a recess in a tibial baseplate, coupling a capture element to the tibial baseplate and/or coupling the hinge post with the bushing within the recess. Thus, the method described above need not be limited in the order of the steps described.

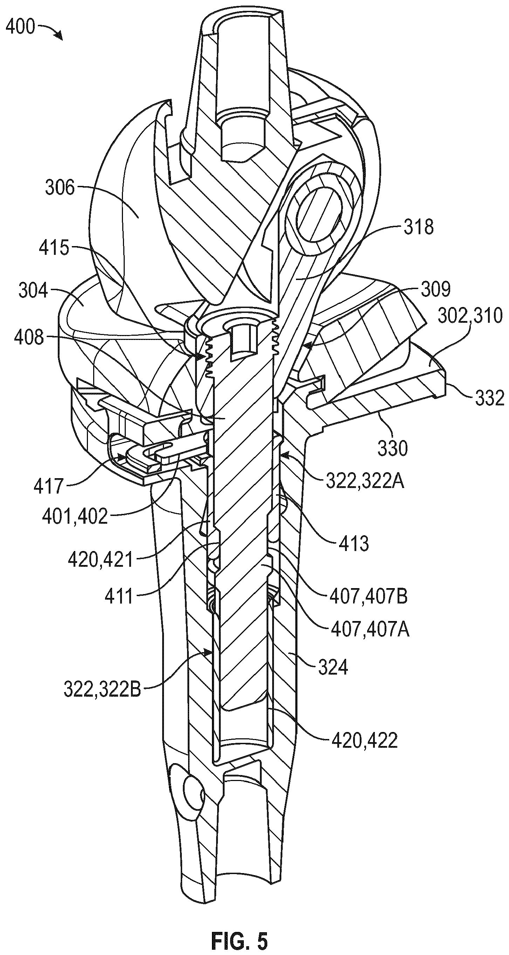

FIG. 5 is a cross-section of a prosthesis assembly 400 having a construction similar to that of the prosthesis assembly 300 described previously. However, the prosthesis assembly 400 differs and includes a capture element 401 comprising a retention clip 402 that limits distraction of the femoral component 306 from the tibial bearing component 304 and the tibial baseplate 302. At least the tibial baseplate 302 can be configured to receive and/or couple with the capture element 401 as further discussed herein.

The cross-section of FIG. 5 shows a distal surface 330 of the tibial baseplate 302 as well as the proximal surface 310 and a periphery 332. The periphery 332 extends around the tibial baseplate 302 between the proximal surface 310 and the distal surface 330. The keel 324 extends distally from the distal surface 330 of the tibial baseplate 302. The keel 324 at least partially defines the recess 322 that receives a hinge post 408 and a bushing 420. The keel 324 can be integral with (monolithic) or can be attached to (e.g. via thread or another mechanical connection mechanism) a remainder of the tibial baseplate 302. The keel 324 can be configured to extend distally and can be shaped to fit in an intermedullary canal of the tibia 106 (FIG. 1) to provide fixation for the tibial baseplate 302.

The distal surface 330 can include features such as threaded apertures for the connection of pegs, augments or other components as known in the art. Distal surface 330 (and other features of the tibial baseplate such as the keel 324) can be made of a porous or highly porous material that facilitates an amount of bone ingrowth. A highly porous biomaterial is useful as a bone substitute and as cell and tissue receptive material. A highly porous biomaterial may have a porosity as low as 30%, 55%, or as high as 70%, 80%, 85%, or 90%. The highly porous material can have an average pore size of between 100 microns and 1000 microns, for example. However, use of the highly porous biomaterial is not contemplated in all examples. For example, material such as bone cement can be utilized as an alternative to the highly porous biomaterial.

An example of such porous or highly porous material is OsseoTi^{®} generally available from Zimmer Biomet, Inc., of Warsaw, Ind. The material can include titanium or titanium alloy and can additionally include other materials. Such material (including a base of relatively less porous or non-porous biocompatible material) can be manufactured using additive manufacturing processes such as laser sintering or the like. OsseoTi^{®} is highly biocompatible, has high corrosion resistance and includes a highly interconnected porous architecture that mimics the porous structure of human cancellous bone, which can enhance bone integration and in-growth. The porous or highly porous material can be manufactured to be layered over or otherwise structured with/on a relatively less porous or non-porous biocompatible material such as titanium, titanium alloy, stainless steel or other material as known in the art.

Another example of such a porous or highly porous material is produced using Trabecular Metal^{™} Technology generally available from Zimmer Biomet, Inc., of Warsaw, Ind. Such a material may be formed from a reticulated vitreous carbon foam substrate which is infiltrated and coated with a biocompatible metal, such as tantalum, by a chemical vapor deposition ("CVD") process in the manner disclosed in detail in U.S. Pat. No. 5,282,861 to Kaplan, the entire disclosure of which is hereby expressly incorporated herein by reference. In addition to tantalum, other metals such as niobium, or alloys of tantalum and niobium with one another or with other metals may also be used. The porous tantalum structure may be made in a variety of densities in order to selectively tailor the structure for particular applications. In particular, as discussed in the above-incorporated U.S. Pat. No. 5,282,861, the porous tantalum may be fabricated to virtually any desired porosity and pore size, and can thus be matched with the surrounding natural bone in order to provide an improved matrix for bone ingrowth and mineralization.

Generally, the porous material structures contemplated can include a large plurality of ligaments defining open spaces there between, with each ligament generally including a core covered by a thin film of metal. The open spaces between the ligaments form a matrix of continuous channels having no dead ends, such that growth of cancellous bone through the porous tantalum structure is uninhibited. The porous or highly porous material may include up to 70%, 85%, or more void space therein. Thus, porous or highly porous material is a lightweight, strong porous structure which is substantially uniform and consistent in composition, and closely resembles the structure of natural cancellous bone, thereby providing a matrix into which cancellous bone may grow to provide fixation of the tibial baseplate to the patient's bone.

FIG. 5 shows the prosthesis assembly 400 can include a hinge post 408 and a bushing 420 that are modified compared with the hinge post 308 and the bushing 320 of the example of FIG. 4. The bushing 420 includes a first piece 421 and a second piece 422. The first piece 421 can be spaced proximally within a proximal portion 322A of the recess 322 from the second piece 422. The second piece 422 is received in a distal portion 322B of the recess 322. Further examples contemplate the first piece 421 and the second piece 422 can be in contact with one another or can be a single component.

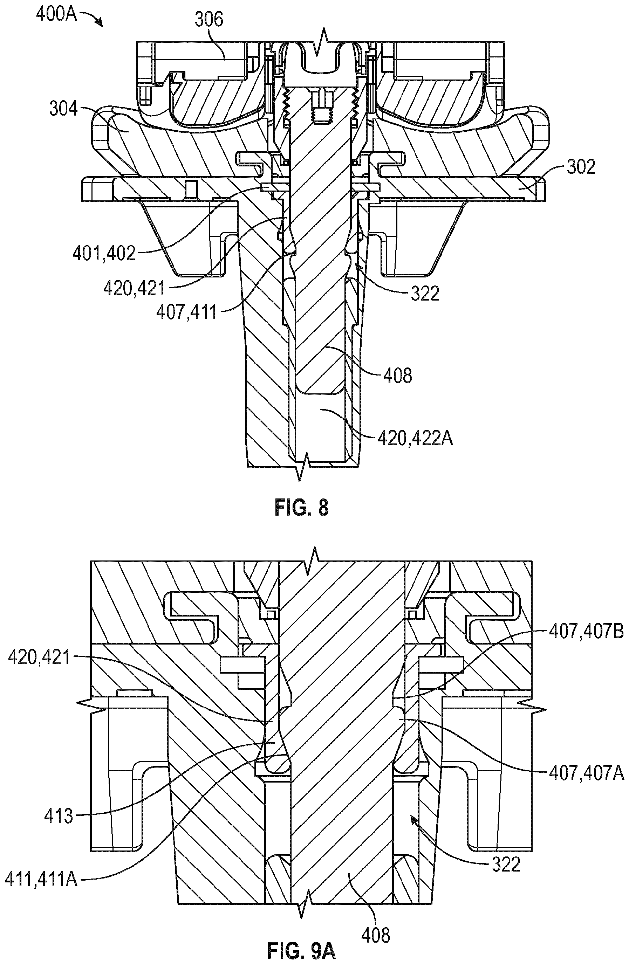

The bushing 420 can be configured to receive at least a portion of the hinge post 408 therein. The hinge post 408 can include engagement features 407. These engagement features 407 can include projections (ribs, tabs, barbs, etc.) and/or apertures (e.g., grooves, holes, recesses, etc.) alone or in combination (a combination of a barb 407A and groove 407B are shown in the example of FIG. 5). The engagement features 407 can be configured to couple with one or more corresponding/mating engagement features 411 of the first piece 421 of the bushing 420. The engagement features 411 can be part of a tabs, arms, feet, prongs or another type of flexible features 413, for example. The flexible features 413 can temporarily flex and deform and can be biased inward to engage the hinge post 408 to create a snap-fit connection between the first piece 421 of the bushing 420 and the hinge post 408 as further illustrated and discussed in FIGS. 8-10. The hinge post 408 and the bushing 420 can be coupled for movement together via engagement of the engagement features 407, 411 when fully seated into a snap-fit connection between the first piece 421 of the bushing 420 and the hinge post 408. This configuration can differ from the construction of the hinge post 308 and the bushing 320 of the example of FIG. 4, which are not coupled for movement together. Thus, the hinge post 308 and the bushing 320 do not include engagement features such as threads. The hinge post 308 can be moveable relative to the bushing 320.

The second piece 422 of the bushing 420 can be inserted into the distal portion 322B and can receive a distal portion of the hinge post 408. The second piece 422 can be spaced from the first piece 421.

FIG. 5 shows the hinge post 408 coupled with the shackle 318 at a proximal end thereof. This can be via a thread connection 415 between the hinge post 408 and the shackle 318 that allows the hinge post 408 and the shackle 318 to be reversibly coupled to and decoupled from one another allowing for change in the configuration of the hinge post (e.g., a change from the hinge post 308 to the hinge post 408, for example, to facilitate limited distraction). The shackle 318 and the hinge post 408 can extend into and be received by the recess 309 of the tibial bearing component 304. The hinge post 308 can have the engagement features 407 arranged along a middle or distal portion of the hinge post 408.

The capture element 401 (the retention clip 402 here) can be insertable and removable into a receptacle 417 within the tibial baseplate 302. The receptacle 417 can be positioned proximal of and adjacent the recess 322 and can communicate with the recess 322. The receptacle 417 can be distal of the proximal surface 310 of the tibial bearing component 302. Thus, the receptacle 417 can be a recessed feature. The first piece 421 of the bushing 420 can have a desired position within the recess 322 relative to the capture element 401 (e.g., can be positioned distal a distance or can be in abutting contact). This position for the first piece 421 of the bushing 420 relative to the capture element 401 can allow for a desired amount of limited distraction between the femoral component 306 and the tibial bearing component 304 and the tibial baseplate 302 prior to engagement of first piece 421 of the bushing 420 with the capture element 401. Although the example of FIG. 5 contemplates that engagement between the bushing 420 and the capture element 401 halts/stops movement to provide for limited distraction of the femoral component 306 relative to the tibial bearing component 304 and the tibial baseplate 302, other examples such as those described in U.S. Provisional Patents both Entitled, ANTI-LUXATION DEVICES FOR A CONSTRAINED PROSTHETIC KNEE, filed on the even day herewith, the entire contents of each of which are incorporated herein by reference, contemplate that engagement features (e.g., barbs, tabs, ribs or other type of projections) that are part of the hinge post and these engagement features engage the capture element 401 to halt/stop distraction of the femoral component 306 relative to the tibial bearing component 304 and the tibial baseplate 302. Thus, the capture element 401 is engaged by at least one of the bushing 420 and/or the hinge post 408 to limit distraction of the femoral component 306 from the tibial bearing component 304 and the tibial baseplate 302.

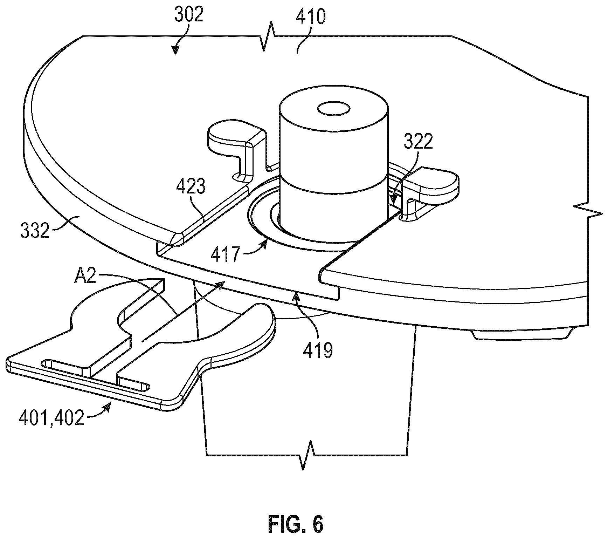

FIG. 6 shows a perspective view of the capture element 401 (the retention clip 402) in the process of being inserted to engage the tibial baseplate 302 via the receptacle 417. The receptacle 417 can have an opening 419 located at the periphery 332 of the tibial baseplate 302. This opening 419 can be at an anterior side, medial side, anterior/medial side, lateral side, anterior/lateral side or other side of the periphery 332, for example.

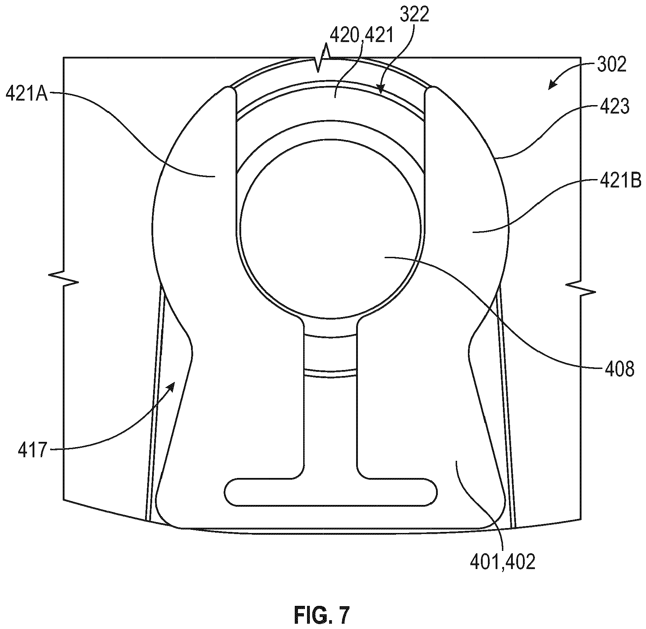

FIG. 7 illustrates that upon full insertion into the receptacle 417, the capture element 401 can be positioned adjacent to or at least partially within the recess 322 of the tibial baseplate 302 such as proximal of the first piece 421 of bushing 420. As shown in both FIGS. 6 and 7, the retention clip 402 can have wings 421A and 421B configured to engage and form an interference fit with walls 423 of the tibial baseplate 302 that define the receptacle 417. The wings 421A and 421B are shaped to receive the hinge post 408 as best illustrated in FIG. 7. However, the wings 421A and 421B can be configured so as not to engage an outer diameter of the hinge post 408 but rather to have a small clearance with the hinge post 408 to allow for limited distraction before the first piece 421 of bushing 420 makes contact with a distal side of the retention clip 402. Thus, the wings 421A and 421B can be configured to allow the hinge post 408 to pass therethrough and extend distally to engage and couple with the bushing 420 as shown in FIG. 5. The retention clip 402 can comprise a component that, when positioned at least partially within the receptacle 417 is coupled with the tibial baseplate 302 (such as via interference fit). Such arrangement for the retention clip 402 can halt/restrict movement of the hinge post 408 upon engagement with the retention clip 402 (via the bushing 420).

FIG. 6 shows part of the tibial baseplate 302, and hence, shows part of the proximal surface 310 and the periphery 332. The proximal surface 310 and periphery 332 can have a particular asymmetry, with respect to a medial/lateral centerline. This shape is designed to maximize tibial coverage for a large proportion of knee-replacement candidates. The asymmetric shape results in a medial compartment of the tibial baseplate being relatively larger than the lateral compartment of the tibial baseplate 302. Maximized coverage of cortical bone facilitates superior support of tibial baseplate 302. A firm, enduring fixation of tibial baseplate 302 to tibia is facilitated by large-area contact between the cortical and cancellous bone of tibia.

FIG. 6 shows the retention clip 402 in the process of being assembled with the tibial baseplate 302 (including by being inserted into the receptacle 417 that is above and/or in communication with the recess 322). The shackle and femoral component are not shown in FIG. 6 for clarity. The bushing 420 (the first piece 421) can be spaced a distance distal of the retention clip 402 upon insertion. The assembly process includes inserting the retention clip 402 into the receptacle 417 that is adjacent to and communicates with the recess 322 and engaging the walls 423. The process includes moving (as indicated by arrow A2) the retention clip 402 generally posterior from the opening 419 toward the recess 322 (e.g., fully into the receptacle 417 to the position of FIGS. 5 and 7).

FIG. 7 additionally illustrates the wings 421A and 421B form a thru hole or other passage for the retention clip 402 as respects the hinge post 408. The passage is configured to allow at least a portion of the hinge post 408 to pass therethrough into a remainder of the recess 322 and to the bushing 420.

FIG. 8 shows a cross-section of a prosthesis assembly 400A including the tibial baseplate 302, part of the tibial bearing component 304, part of the femoral component 306, the capture element 401 (retention clip 402), the first piece 421 of the bushing 420 and the hinge post 408. However, the example of FIG. 8 has a slightly modified shape for the second piece 422A as compared with the example of FIG. 5. Additionally, the first piece 421 is in abutting contact with the retention clip 402 unlike the example of FIG. 5.

As with the example of FIG. 5, the example of FIG. 8 includes the first piece 421 is configured to create a snap-fit or other type of coupling engagement with the hinge post 408 with interaction of the engagement features 407, 411.

FIGS. 9A-9C show a process of insertion of the hinge post 408, insertion of the first piece 421 of the bushing 420 and coupling of the hinge post 408 with the first piece 421 of the bushing 420. FIGS. 9A-9C illustrates the process of eventual coupling of the engagement features 407 and 411.

As discussed previously, the engagement features 407 can include the combination of the barb 407A and the groove 407B. The engagement features 407 can be configured to couple with one or more corresponding/mating engagement features 411 of the first piece 421 of the bushing 420. The engagement features 411 can be projections 411A each having a tapered, ramped or chamfered surface. The projections 411A (and the engagement features 411) can be part of a tab, arm, foot, prong or another flexible features 413 of the first piece 421, for example.

FIG. 9A shows initial insertion of the hinge post 408 and the first piece 421 of the bushing 420 into the recess 322. The location the hinge post 408 and the first piece 421 in FIG. 9A can be proximal of the fully inserted position within the recess 322 shown in FIG. 8. In FIG. 9A, the barb 407A can be received proximal of the engagement features 411. Specifically, the barb 407A can be proximal of the projections 411A. The barb 407A can be received inward of the flexible features 413 of the first piece 421. Projections 411A can extend inward toward a centerline axis of the hinge post 408 and toward a center of a central thru hole of the first piece 421 of the bushing 420 from a remainder of the flexible features 413.

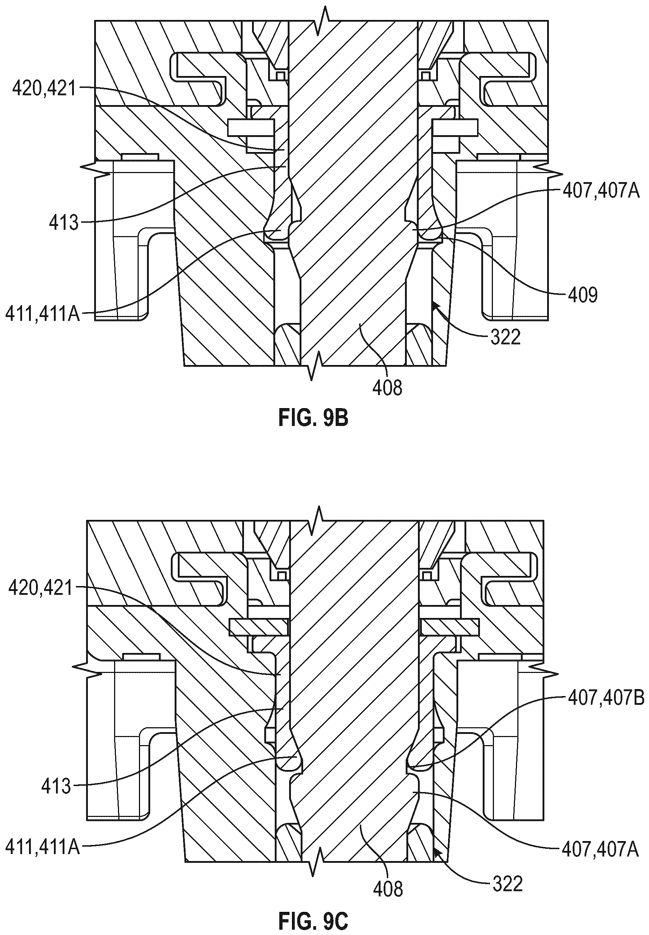

FIG. 9B shows the hinge post 408 moved distal relative to the recess 322 and the first piece 421 of the bushing 420. This movement of the hinge post 408 can bring the barbs 407A distally down to engage the engagement features 411 (the projections 411A). This engagement can cause the flexible features 413 to temporarily flex and deform outward into a groove 409 or other feature along an outer wall of the recess 322. The groove 409 can receive the outer portion or tip of the flexible feature 413 allowing for passage of the hinge post 408 and the barbs 407A to a position distal of the engagement features 411 (the projections 411A).

FIG. 9C shows the first piece 421 of the bushing 420 and the hinge post 408 moved distally from the position of FIG. 9B to a fully inserted position such as previously shown in FIG. 8. FIG. 9C shows that after passage of the barbs 407A past the projections 411A, the flexible features 413 can be biased inward to engage the projections 411A with the hinge post 408 at the groove 407B to create a snap-fit connection between the first piece 421 of the bushing 420 and the hinge post 408. Once engaged in the manner of FIG. 9C, the hinge post 408 and the bushing 420 can be coupled for movement together. This is via engagement of the engagement features 407, 411 when fully seated into the snap-fit connection shown in FIG. 9C.

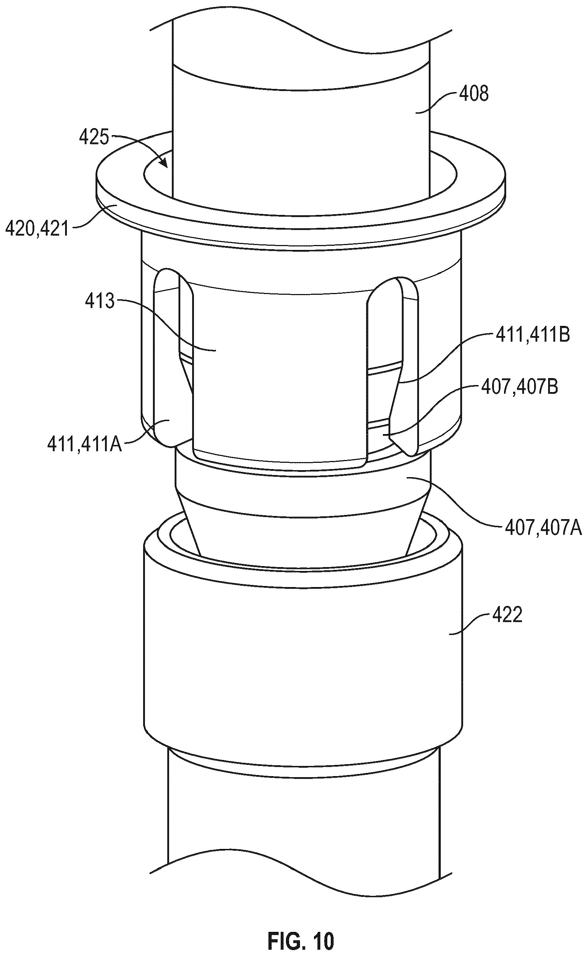

FIG. 10 shows a perspective view of the first piece 421, the second piece 422 and the hinge post 408 shown in isolation in the snap-fit connection position of FIGS. 8 and 9C. FIG. 10 illustrates the hinge post 408 with the engagement features 407 (the barb 407A and the groove 407B). The first piece 421 of the bushing 420 can include a thru hole 425 configured to receive at least a portion of the hinge post 408. FIG. 10 also shows the engagement features 407 can be configured to couple with one or more corresponding/mating engagement features 411 of the first piece 421 of the bushing 420. The engagement features 411 can be the projections 411A each having a tapered, ramped or chamfered surface 411B. The projections 411A (and the engagement features 411) can be part of the flexible features 413 of the first piece 421. The flexible features 413 can be spaced apart circumferentially from one another. The engagement features 411 (the projections 411A) can extend inward toward a centerline of the first piece 421 of the bushing 420 and the hinge post 408 and can be shaped to engage the groove 407B.

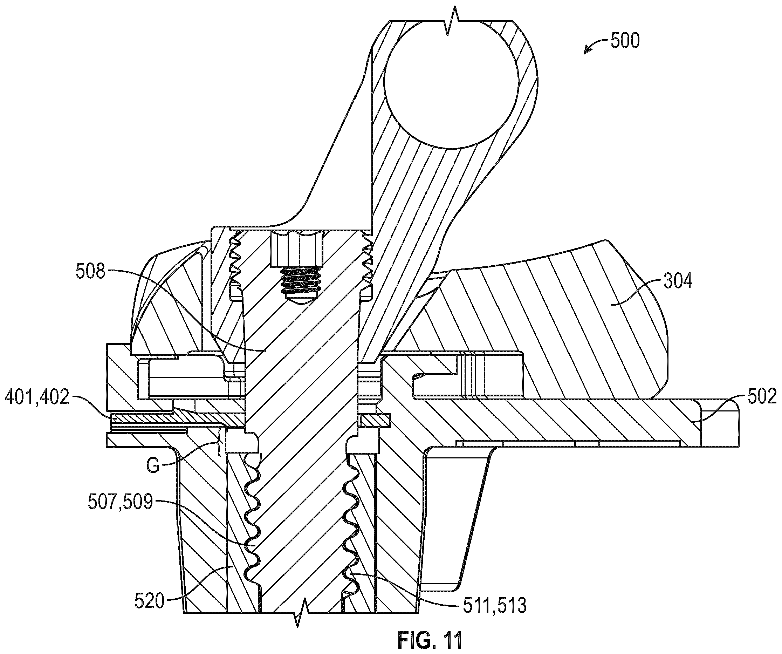

FIG. 11 is a cross-section of another example of a prosthesis assembly 500 that utilizes the capture element 401 (the retention clip 402). The capture element 401 can have the construct described previously. FIG. 11 additionally shows further examples of a hinge post 508 and a bushing 520. The hinge post 508 can have the engagement features 507 (here threads 509) arranged along a middle portion of the hinge post 508. These engagement features 507 can couple with corresponding engagement features 511 (threads 513) of the bushing 520. The threads 509 and threads 513 of the bushing 520 can be rope threads, knuckle threads, ACME threads or other types of threads as known in the art. In some examples, the threads 509 can differ slightly in pitch or another geometric aspect from the threads 513 so as to create a slight interference. This slight interference can draw the bushing 520 to a desired position within a recess 522 relative to the capture element 401. This position for the bushing 520 relative to the capture element 401 can allow for a desired amount of limited distraction between the femoral component (not shown) and the tibial bearing component 304 and a tibial baseplate 502 prior to engagement of the bushing 520 with the capture element 401. For example, the bushing 520 can move to create a desired gap G between the bushing 520 and the capture element 401. The size of the gap G can dictate the amount of distraction of the femoral component (not shown) from the tibial bearing component 304 and the tibial baseplate 302. Distraction can be limited (halted) by contact between the bushing 520 (or hinge post 408 in other examples) with the capture element 401 upon movement of the bushing 520 with the hinge post 508 proximally.

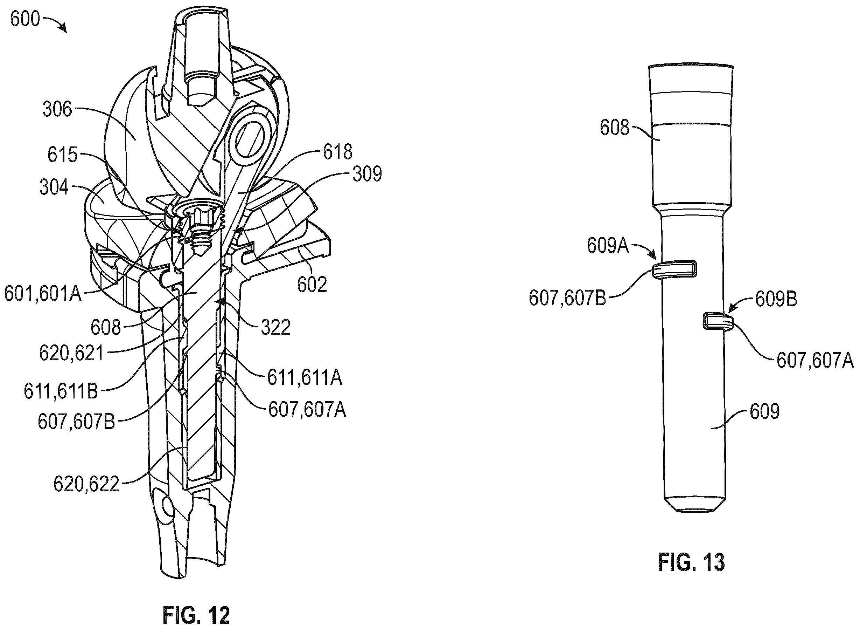

FIG. 12 shows a cross-section of a prosthesis assembly 600 including a tibial baseplate 602, part of the tibial bearing component 304 and part of the femoral component 306. The example of FIG. 12 includes a capture feature 601 such as a nut 601A configured to couple with threading 615 to a modified shackle 618. A hinge post 608 can be located distal of and is captured by so as to be retained by the nut 601A.

A bushing 620 can be modified from the examples shown previously. The bushing 620 can include a first piece 621 and a second piece 622. The hinge post 608 can include engagement features 607 such as two or more barbs 607A and 607B. The barb 607A can be distal of the barb 607B. The first piece 621 of the bushing 620 can include engagement features 611 such as two or more barbs 611A and 611B. The barb 611A can be distal of the barb 611B.

The hinge post 608 can be inserted down into the bushing 620 and the recess 322. However, the two or more barbs 607A and 607B and the two or more barbs 611A and 611B require that the hinge post 408 be clocked (rotated) while being inserted into the fully engaged position of FIG. 12. This rotation and distal insertion of the hinge post 608 allows the barb 607A to pass to a position distal of the barb 611A and the barb 607B to pass to a position distal of the barb 611B. The barb 607A can abut the barb 611A and the barb 607B can abut the barb 611B (although such relationship is not required if some gap is desired). Proximal movement of the hinge post 608 such as occurs during limited distraction can be retrained/stopped by engagement of the engagement features 607 and 611 (the engagement of the barb 611A and the barb 607A and the barb 611B and the barb 607B).

FIG. 13 shows a perspective view of the hinge post 608 with the engagement features comprising the two or more barbs 607A and 607B. The barbs 607A and 607B can extend from an outer diameter 609 of the hinge post 608. The barbs 607A and 607B can extend around less than a full circumference of the hinge post 608 (and indeed less than half the circumference), for example. The barb 607A can be distal of the barb 607B. The barb 607A can be positioned on a first half 609A of the outer diameter 609. The barb 607B can be positioned on a second half 609B of the outer diameter 609.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "generally" "substantially" "about" mean within 15 percent of the value provided (±). The terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other examples can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above detailed description, various features can be grouped together to streamline the disclosure. This should not be interpreted as intending that an unclaimed disclosed feature is essential to any claim. Rather, inventive subject matter can lie in less than all features of a particular disclosed example. Thus, the following claims are hereby incorporated into the detailed description as examples or embodiments, with each claim standing on its own as a separate example, and it is contemplated that such examples can be combined with each other in various combinations or permutations. The scope of the invention should be determined with reference to the appended claims, along with the full scope of equivalents to which such claims are entitled.

## Claims

1. A prosthesis assembly for a constrained knee comprising:
a femoral component;
a tibial bearing component engaged by the femoral component;
a tibial baseplate having a distal surface, a proximal surface opposite the distal surface and facing the tibial bearing component, a periphery extending between the proximal surface and the distal surface and a keel extending distally from the distal surface;
a hinge post coupled to the femoral component and at least partially received in a recess in the tibial baseplate; and
a bushing received in a recess in the tibial baseplate, wherein the hinge post is at least partially received by the bushing, and wherein the bushing includes one or more engagement features configured to couple the bushing to the hinge post.

2. The prosthesis assembly of claim 1, further comprising a capture element coupled to one of a shackle or the tibial baseplate, wherein the capture element is engaged by at least one of the bushing or the hinge post to limit distraction of the femoral component from the tibial bearing component and the tibial baseplate.

3. The prosthesis assembly of claim 2, wherein at least one of: the capture element is spaced a distance proximal of the bushing, and wherein the distance allows for a limited degree of distraction of the femoral component from the tibial bearing component and tibial baseplate or the capture element is proximal to and abuts a proximal end of the bushing and thereby entirely limits distraction of the femoral component from the tibial bearing component and tibial baseplate.

4. The prosthesis assembly of any one of claims 2-3, wherein the capture element comprises one of a nut configured to thread to the shackle or a retention clip insertable into a receptacle within the tibial baseplate.

5. The prosthesis assembly of claim 4, wherein the receptacle communicates with the recess and has an opening at the periphery of the tibial baseplate configured to receive the retention clip.

6. The prosthesis assembly of any one of claims 1-5, wherein the bushing is configured to form an snap-fit to capture the hinge post.

7. The prosthesis assembly of claim 6, wherein, during insertion of the hinge post into the recess, the bushing is configured to temporarily deflect outward into an aperture in an inner wall that forms at a portion of the recess of the tibial baseplate.

8. The prosthesis assembly of claim 7, wherein the hinge post includes one or more projections configured to force the bushing to temporarily deflect outward into the aperture.

9. The prosthesis assembly of one of claim 7 or 8, wherein the hinge post includes a groove configured to receive one or more inward extending projections of the bushing, wherein the one or more inward extending projections extend toward a center of the hinge post.

10. The prosthesis assembly of any one of claims 1-9, wherein the bushing includes a first bushing configured to receive a proximal portion of the hinge post within a proximal portion of the recess and a second bushing configured to receive a distal portion of the hinge post within a portion of distal portion of the recess, and wherein the first bushing is spaced from the second bushing.

11. The prosthesis assembly of any one of claims 1-10, wherein the hinge post includes one or more retention features configured to be engaged by the bushing to limit distraction of the femoral component from the tibial bearing component and tibial baseplate.

12. The prosthesis assembly of claim 1, wherein the hinge post is coupled to the bushing by first threads that couple with second threads of the bushing, and wherein coupling of the first threads with the second threads positions the bushing a desired location with respect to a capture element.

13. A method of assembling a prosthesis assembly for a constrained knee, the method comprising:
coupling a hinge post with a femoral component;
inserting a bushing while receiving at least a portion of the hinge post into a recess in a tibial baseplate;
coupling a capture element to the tibial baseplate;
coupling the hinge post with the bushing within the recess;
coupling a tibial bearing component to the tibial baseplate; and
mounting the femoral component on the tibial bearing component.

14. The method of claim 13, wherein coupling the hinge post with the bushing within the recess of the tibial baseplate includes temporarily deflecting the bushing outward to allow for passage of the hinge post along the bushing and engaging the bushing with one or more retention features of the hinge post.

15. The method of any one of claims 13-14, wherein coupling the capture element to the tibial baseplate includes positioning the capture element adjacent the recess of the tibial baseplate and includes inserting the capture element through an opening in a periphery of the tibial baseplate into a receptacle that is adjacent to and communicates with the recess.
